(19) Europäisches Patentamt
European Patent Office
Office européen des brevets

(11) Publication number: **0 070 618**
**A1**

# (12) EUROPEAN PATENT APPLICATION

(21) Application number: **82302891.5**

(22) Date of filing: **04.06.82**

(51) Int. Cl.³: **C 07 D 301/12**, C 07 D 301/19, C 07 D 303/14

(30) Priority: **19.06.81 GB 8118895**

(43) Date of publication of application: **26.01.83**
**Bulletin 83/4**

(84) Designated Contracting States: **BE CH DE FR GB IT LI NL**

(71) Applicant: **IMPERIAL CHEMICAL INDUSTRIES PLC, Imperial Chemical House Millbank, London SW1P 3JF (GB)**

(72) Inventor: **Jackson, Reginald, 74 Hayhurst Avenue, Middlewich Cheshire (GB)**
Inventor: **John, Glyn Rhys, 62 Wharfedale Greenhouse Farm, Runcorn Cheshire (GB)**

(74) Representative: **Stephenson, Kenneth et al, Imperial Chemical Industries PLC Legal Department: Patents Thames House North Millbank, London SW1P 4QG (GB)**

(54) **Asymmetric epoxidation process.**

(57) A method for the preparation of a chiral epoxy alcohol which comprises reacting a primary allylic or homoallylic alcohol under anhydrous conditions with at least a stoichiometric amount of hydrogen peroxide or an alkyl or aryl hydroperoxide in the presence of a $Ti^{IV}$ complex and a chiral amino alcohol having at least one centre of chirality.

EP 0 070 618 A1

TITLE MODIFIED see front page

0070618

CHEMICAL PROCESS

This invention relates to a chemical process and more particularly to a highly selective method for the asymmetric epoxidation of an allylic or homoallylic alcohol.

Methods have been proposed for the asymmetric epoxidation of olefinic compounds with the object of producing chiral epoxides which are of great potential value in the fields of biochemistry and synthetic chemistry, both scientifically and industrially. In general, methods involving metal-catalysed epoxidation with alkyl hydroperoxides in the presence of a wide selection of chiral ligands have not been completely satisfactory, the optical inductions achieved being somewhat disappointing.

More recently, K. B. Sharpless et al have reported (J. Amer. Chem. Soc., Vol. 102, 5974, 1980) a method of making optically active epoxides from primary allylic alcohols which involves the use of tert-butyl hydroperoxide and titanium tetraisopropoxide in the presence of (+) or (-) diethyl tartrate, other closely related reagents being relatively ineffective. It has now surprisingly been found that these asymmetric transformations

BAD ORIGINAL

can be effected under a wider range of reaction conditions if diethyl tartrate is replaced by a chiral amino alcohol. This finding is in contrast to the previously reported (S. Yamada, T Mashiko and S Terashima, J Amer. Chem. Soc., Vol. 99, 1988, 1977) use of amino alcohols ((-)-N-alkyl-ephedrines) in the molybdenum-based asymmetric epoxidation of allylic alcohols where the highest optical induction obtained was 33%.

Accordingly, the present invention provides a method for the preparation of a chiral epoxy alcohol which comprises reacting a primary allylic or homoallylic alcohol under anhydrous conditions with at least a stoichiometric amount of hydrogen peroxide or an alkyl or aryl hydroperoxide in the presence of a $Ti^{IV}$ complex and a chiral amino alcohol having at least one centre of chirality.

Primary allylic alcohols which may be employed in the method of the invention include compounds of the formula:

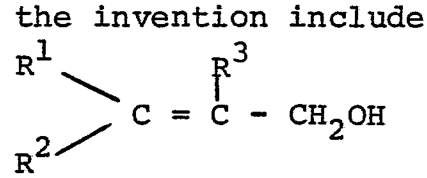

wherein each of $R^1$, $R^2$ and $R^3$, independently, represents hydrogen or an optionally substituted alkyl or aryl radical. Corresponding homoallylic alcohols may also be used.

As specific examples of suitable alcohols there may be mentioned allyl alcohol, crotyl alcohol, cinnamyl alcohol, 2-phenylcinnamyl alcohol and geraniol.

Hydroperoxides which may be used in the method of the invention include <u>tert</u>-butyl hydroperoxide and cumene hydroperoxide.

$Ti^{IV}$ complexes which may be used include, in particular, the alkoxides, for example titanium tetraisopropoxide. If desired, the $Ti^{IV}$ complex may be formed <u>in situ</u> from other titanium compounds in known manner.

The chiral amino alcohol used in the method of the invention contains an amino group which is preferably a primary or secondary amino group, a hydroxyl group

which is preferably a primary or secondary hydroxyl group and at least one centre of chirality. The amino and hydroxyl groups are preferably attached to adjacent carbon atoms.

Specific examples of suitable amino alcohols include aliphatic compounds such as (+)-L-2-amino-1-propanol, (-)-D-2-amino-1-butanol and (-)-L-2-amino-3-methyl-1-butanol, aryl substituted aliphatic compounds such as (-)-L-2-amino-3-phenyl-1-propanol and (-) or (+)-ephedrine and heterocyclic compounds such as (+)-L-prolinol.

In carrying out the method of the invention, the peroxide or hydroperoxide is used in an amount that is at least equivalent to the allylic or homoallylic alcohol and it is advantageous to use approximately two equivalents thereof for each equivalent of the alcohol.

The $Ti^{IV}$ complex and the chiral amino alcohol are generally used in substantially equimolar amounts. The amounts of each to be used relative to the allylic (or homoallylic) alcohol depends upon the reactivity of the latter. In the case of the more reactive alcohols containing electron-donating substituents, the $Ti^{IV}$ complex and the amino alcohol can be used in catalytic amounts, for example 0.1 mole of each per mole of allylic alcohol. With the less reactive allylic alcohols, it is necessary to use substantially stoichiometric amounts of the $Ti^{IV}$ complex and the amino alcohol relative to the allylic alcohol for the reaction to be completed within a reasonable time.

The reaction is conveniently performed in an inert solvent, that is to say in a solvent which is inert with respect to the reaction mixture components. Suitable solvents include hydrocarbons, for example toluene, halogenated hydrocarbons such as methylene dichloride, 1,2-dichloroethane and chloroform, and especially polar aprotic solvents such as acetone and ethyl acetate which can lead to improved optical yields.

The most suitable reaction temperature depends to some extent on the particular reactants being used,

especially in respect of the degree of optical selectivity obtained. A temperature in the range of -50°C to 50°C is generally suitable, the range of -30° to 20°C being preferred. A significant advantage for the method of the invention over the prior art method referred to above is the convenience of being able to carry out the reaction at normal ambient temperatures.

The reaction is continued until it has reached substantial completion and generally takes a few hours depending upon the particular reactants and reaction temperature being employed. The reaction product may then be isolated using completely conventional methods.

The method of the invention results in a high yield of epoxy alcohol, yields of approximately 90% being common. For a given allylic alcohol, the optical yield, that is the degree of enantioselectivity produced by the method of the invention, varies according to the particular amino alcohol employed. With some amino alcohols, the excess of one epoxy alcohol enantiomer over a 50-50 mixture of enantiomers can be 60-90% or even higher. The optical yield can be determined by a standard chiral shift n.m.r. spectroscopy method.

The invention is illustrated but not limited by the following Examples.

Example 1

50 ml of dry reagent grade 1,2-dichloroethane was poured into a 100 ml 2-neck round-bottom flask with magnetic stirrer and cooled to -20°C in an acetone/dry ice bath. After thorough purging with nitrogen gas, 1.42g (5 mmol) of titanium tetraisopropoxide and 0.375g (5 mmol) of (+)-L-2-amino-propanol were added to the flask via a serum cap and allowed to stir for 15 minutes. After the addition of 1.05g (5 mmol) of 2-phenyl-cinnamyl alcohol and stirring for a further 15 minutes, 3.2 ml of a dichloroethane solution (0.28g TBHP/ml) containing

10 mmol of anhydrous tert-butyl hydroperoxide were added and the reaction allowed to proceed at -20°C for approximately 17 hours, after which time the epoxidation was substantially complete as judged by T.L.C. monitoring.

20 ml of 10% aqueous tartaric acid solution were added to the mixture which was stirred and allowed to warm up to room temperature (ca. 20 minutes). After separation of the organic layer and acetylation with 5 ml of acetic anhydride/5 ml of pyridine, H.P.L.C. methods were used to isolate 1.16g (87%) of the product oil, the epoxy acetate from 2-phenyl cinnamyl alcohol. Analysis of the product by $Eu(hfbc)_3$ chiral shift n.m.r. spectroscopy gave an otpical yield of >60% $^e/e$ (enantiomeric excess).

Example 2

The procedure described in Example 1 was repeated except that the (+)-L-2-aminopropanol was replaced by 0.825g (5 mmol) of (+) or (-) ephedrine.

1.18g (88%) of the epoxy acetate product was isolated from the reaction mixture, having an optical yield of >72% $^e/e$ by $Eu(hfbc)_3$ shift n.m.r. spectroscopy.

Example 3

The procedure described in Example 1 was repeated except that the (+)-L-2-aminopropanol was replaced by 0.505g (5 mmol) of (+)-L-2-pyrrolidine methanol (i.e. (+)-prolinol). 1.27g (90%) of the epoxy acetate product was isolated, and exhibited an optical yield of >90% by $Eu(hfbc)_3$ shift n.m.r. spectroscopy.

Example 4

The procedure described in Example 1 was repeated except that the (+)-L-2-aminopropanol was replaced by 0.825g (5 m mol) of (+) ephedrine and the 1,2-dichloroethane was replaced by acetone. The epoxy acetate product exhibited an optical yield of >90% by $Eu(hfbc)_3$ shift nmr spectroscopy.

CLAIMS

1. A method for the preparation of a chiral epoxy alcohol which comprises reacting a primary allylic or homoallylic alcohol under anhydrous conditions with at least a stoichiometric amount of hydrogen peroxide or an alkyl or aryl hydroperoxide in the presence of a $Ti^{IV}$ complex and a chiral amino alcohol having at least one centre of chirality.

2. A method according to claim 1 wherein the primary allylic alcohol is a compound of the formula:

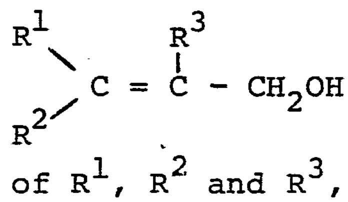

wherein each of $R^1$, $R^2$ and $R^3$, independently, represents hydrogen or an optionally substituted alkyl or aryl radical.

3. A method according to claim 2 wherein the primary allylic alcohol is 2-phenylcinnamyl alcohol.

4. A method according to any one of the preceding claims wherein the $Ti^{IV}$ complex is an alkoxide.

5. A method according to claim 4 wherein the $Ti^{IV}$ complex is titanium tetraisopropoxide.

6. A method according to any one of the preceding claims wherein the chiral amino alcohol has an amino group and a hydroxyl group attached to adjacent carbon atoms.

7. A method according to claim 6 wherein the chiral amino alcohol is (+)-L-2-amino-1-propanol, (-) or (+)-ephedrine or (+)-L-prolinol.

0070618

European Patent Office

# EUROPEAN SEARCH REPORT

Application number

EP 82 30 2891

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | JOURNAL OF THE AMERICAN CHEMICAL SOCIETY, vol.99, no.6, March 16, 1977, Columbus, Ohio (US) S. YAMADA et al.: "(Acetylacetonato)[(-)-N-alkylephedrinato]dioxomolybdenum, a new class of chiral chelate complexes which catalyze asymmetric epoxidation of allylic alcohol", pages 1988-1990 * the whole article * --- | 1-7 | C 07 D 301/12<br>C 07 D 301/19<br>C 07 D 303/14 |
| Y | CHEMICAL ABSTRACTS, vol.93, no.20, November 17, 1980, page 411, abstract no.192718e, Columbus, Ohio (US) T. MASHIKO et al.: "Stereochemical studies. LV.Synthetic studies on chiral molybdenum (VI) complexes as asymmetric epoxidation catalysts" & Yakugaku Zasshi 1980, 100(3), 319-27 --- | 1-7 | TECHNICAL FIELDS SEARCHED (Int. Cl. 3)<br>C 07 D 301/00<br>C 07 D 303/00 |
| Y | CHEMICAL ABSTRACTS, vol.78, no.10, March 12, 1973, page 567, abstract no.66354k, Columbus, Ohio (US) E.C. ALYEA et al.: "Five-coordinate tris(alkoxy)titanium(IV) complexes" & Inorg. Nucl. Chem. Lett. 1973, 9(1), 69-74 --- -/- | 1-7 | |

The present search report has been drawn up for all claims

| Place of search | Date of completion of the search | Examiner |
|---|---|---|
| THE HAGUE | 02-09-1982 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0070618

European Patent Office

# EUROPEAN SEARCH REPORT

Application number

EP 82 30 2891

Page 2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| Y | CHEMICAL ABSTRACTS, vol.84, no.18, May 3, 1976, page 577, abstract no.129865z, Columbus, Ohio (US) A.M. GOLUB et al.: "Mixed coordination compounds of titanium(IV)with amino alcohols and anions of inorganic acids" & Koord. Khim. 1976, 2(2), 205-12 --- | 1-7 | |
| D,Y | JOURNAL OF THE AMERICAN CHEMICAL SOCIETY, vol.102, no.18, August 27, 1980, American Chemical Society, Columbus, Ohio (US) T. KATSUKI et al.: "The first practical method for asymmetric epoxidation", pages 5974-5976 * the whole article * ----- | 1-7 | TECHNICAL FIELDS SEARCHED (Int. Cl. 3) |

The present search report has been drawn up for all claims

| Place of search | Date of completion of the search | Examiner |
|---|---|---|
| THE HAGUE | 02-09-1982 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82